# EUROPEAN PATENT SPECIFICATION

(11) **EP 1 812 115 B1**
(45) Date of publication and mention of the grant of the patent: **15.05.2013**
(21) Application number: 05805163.2
(22) Date of filing: 21.10.2005
(51) Int. Cl.: A61K 51/12, A61K 9/127, A61K 31/704, A61P 35/00, A61K 103/30, A61K 103/40, A61K 103/00, A61K 101/02

(54) **Liposomes enclosing a radionuclide and doxorubicin for combination therapy**
Liposome, die ein Radionuklid und Doxorubicin einkapseln, und deren Verwendung in der Kombinationstherapie
Liposomes encapsuant un radionucléide et la doxorubicine pour une thérapie combinée

(30) Priority: 22.10.2004 GB 0423565
(43) Date of publication of application: 01.08.2007
(73) Proprietor: Algeta ASA, 0884 Oslo (NO)
(72) Inventor: LARSEN, Roy, N-0884 Oslo (NO); JONASDOTTIR, Thora Johanna, N-481 Hagan (NO)
(74) Representative: Goddard, Christopher Robert
(86) International application number: PCT/GB2005/004074
(87) International publication number: WO 2006/043083

(56) References cited:
- WO-A-01/60417
- WO-A2-2004/004635
- US-A1- 2003 175 206
- HENRIKSEN G ET AL: "Sterically stabilized liposomes as a carrier for alpha-emitting radium and actinium radionuclides" NUCLEAR MEDICINE AND BIOLOGY, ELSEVIER, NY, US, vol. 31, no. 4, May 2004 (2004-05), pages 441-449, XP004503655 ISSN: 0969-8051
- SOFOU STAVROULA ET AL: "Engineered liposomes for potential alpha-particle therapy of metastatic cancer." JOURNAL OF NUCLEAR MEDICINE, vol. 45, no. 2, February 2004 (2004-02), pages 253-260, XP002402444 ISSN: 0161-5505
- DAVIES CATHARINA DE L ET AL: "Radiation improves the distribution and uptake of liposomal doxorubicin (Caelyx) in human osteosarcoma xenografts." CANCER RESEARCH, vol. 64, no. 2, 15 January 2004 (2004-01-15), pages 547-553, XP002402474 ISSN: 0008-5472
- KOULOULIAS V E ET AL: "Liposomal doxorubicin in conjunction with reirradiation and local hyperthermia treatment in recurrent breast cancer: A Phase I/II trial" CLINICAL CANCER RESEARCH 2002 UNITED STATES, vol. 8, no. 2, 2002, pages 374-382, XP002402475 ISSN: 1078-0432
- KOUKOURAKIS MICHAEL I ET AL: "High intratumoral accumulation of stealth liposomal doxorubicin in sarcomas: Rationale for combination with radiotherapy" ACTA ONCOLOGICA (STOCKHOLM), vol. 39, no. 2, 2000, pages 207-211, XP002402476 ISSN: 0284-186X
- KOUKOURAKIS M I ET AL: "Liposomal doxorubicin and conventionally fractionated radiotherapy in the treatment of locally advanced non-small-cell lung cancer and head and neck cancer" JOURNAL OF CLINICAL ONCOLOGY 1999 UNITED STATES, vol. 17, no. 11, 1999, pages 3512-3521, XP002402477 ISSN: 0732-183X
- CATTEL L ET AL: "From conventional to stealth liposomes a new frontier in cancer chemotherapy" TUMORI 2003 ITALY, vol. 89, no. 3, 2003, pages 237-249, XP008069891 ISSN: 0300-8916
- JONASDOTTIR T J ET AL: "First in vivo evaluation of liposome-encapsulated <223>Ra as a potential alpha-particle-emitting cancer therapeutic agent" ANTICANCER RESEARCH 2006 GREECE, vol. 26, no. 4 B, 2006, pages 2841-2848, XP008069893 ISSN: 0250-7005
- SRIVASTAVA S ET AL: "RECENT ADVANCES IN RADIONUCLIDE THERAPY", SEMINARS IN NUCLEAR MEDICINE, GRUNE AND STRATTON,, ORLANDO, FL, US, vol. 31, no. 4, 1 October 2001 (2001-10-01), pages 330-341, XP008023366, ISSN: 0001-2998, DOI: DOI:10.1053/SNUC.2001.27043

## Description

The present invention relates to liposome formulations and in particular to liposomes for use in alpha-emitting endo-radionuclide combination therapy.

The treatment of hyperplastic or neoplastic diseases such as cancers frequently requires the administration of cytotoxic agents in order to selectively eliminate cells and cell populations showing uncontrolled or undesirable growth patterns. In the case of localised neoplasms, such as solid tumours, surgery may be used to remove the bulk mass of undesirable cells but increasingly this is being used in combination with local, regional and/or systemic cytotoxic treatment in order to minimise both the risk of recurrence and the amount of tissue resected.

In the case of dispersed neoplastic diseases, such as metastatic cancers, surgery cannot generally be used as the sole mode of treatment and regional or systemic cytotoxic agents will typically be a major part of the therapeutic regime. There is therefore a considerable ongoing need for new and improved cytotoxic systems, particularly for the treatment of neoplastic disease.

For many years, beta-emitting radionuclides have been used as cytotoxic agents in radiopharmaceuticals for cancer therapy. In recent years, however, efforts have also been made to utilize alpha-emitters in anti-tumour agents.

Alpha-emitters have several features which distinguish them from beta-emitters and potentially provide for increased effectiveness in therapy. These include their higher energies and shorter ranges in tissues.

The radiation range of typical alpha emitters in physiological surroundings is generally less than 100 micrometers, the equivalent of only a few cell diameters. This makes these sources well suited for treatment of tumours including micrometastases because little of the radiated energy will pass beyond the target cells, and thus damage to the surrounding healthy tissue can be minimised. In contrast, a beta particle has a range of 1 mm or more in water.

The energy of alpha-particle radiation is also high compared to beta particles, gamma rays and X-rays, typically being 5-8 MeV, or 5 to 10 times that of a beta particle and 20 or more times the energy of a gamma ray. Thus, this deposition of a large amount of energy over a very short distance gives alpha radiation an exceptionally high linear energy transfer (LET), when compared to gamma or beta radiation. This explains the exceptional cytotoxicity of alpha-emitting radionuclides and also imposes stringent demands on the level of control and study of radionuclide distribution necessary in order to avoid unacceptable side effects.

A further factor which renders certain alpha-emitting radionuclides both highly suitable as cytotoxic agents and highly challenging as agents for accurate targeting *in vivo* is the properties of their daughter nuclides. In particular, many alpha-emitting radionuclides form part of a decay chain of several alpha and/or beta-transformations between the initial nuclide and a stable daughter isotope. Where any daughter nuclide is also an alpha-emitter, this nuclide must also be targeted and controlled if it is not to cause undesirable toxic effects by accumulating in healthy tissue. Unfortunately, both physical and chemical factors make this control very difficult to achieve.

When a radionuclide decays, the daughter nuclide generated is often chemically quite different from the original isotope. Radium-224, for example, is an alpha emitter but is chemically an alkaline earth metal adopting the 2+ oxidation state. The immediate product of ²²⁴Ra alpha-decay is, however, radon-220, which is a noble gas. Consequently, whatever co-ordination or chelation effects may have previously been stably holding the radium parent ion in a controlled, targeting agent, are unlikely to maintain control over the radon daughter, which may thus diffuse away and decay elsewhere in the body. Obviously, a whole chain of further decay events may follow without any control over where in the body these events take place.

The physical effect of an alpha decay upon the resulting daughter nuclide can also be quite dramatic. The helium nucleus of an alpha particle has a relative atomic mass of 4 and is typically emitted at around 2% of the speed of light. Evidently this imparts a considerable energy to the resulting daughter nucleus which, by simple conservation of momentum, might be expected to recoil at in excess of 100 Km/s. Evidently, the combined destructive force of the very high speed alpha particle and the recoil imparted to the massive daughter nucleus can break chemical bonds, propel the nuclei out of chelation and cause considerable problems in any attempt to control the fate of nuclei further down in the nuclear decay chain. As a result, very few methods have been provided by which alpha decaying nuclei may be utilised in therapy because of the difficulties of maintaining control over the subsequent decays. The decay chains of some alpha emitting isotopes which might be useful in therapy are shown below.

**Table 1**

| Nuclide | Decay mode | Mean particle energy (MeV) | Half-life |
|---|---|---|---|
| ²²⁷Th | α | 6.02 | 18.72 days |
| ²²³Ra | α | 5.78 | 11.43 days |
| ²¹⁹Rn | α | 6.88 | 3.96 seconds |
| ²¹⁵Po | α | 7.53 | 1.78 ms |
| ²¹¹Pb | β | 0.45 | 36.1 minutes |
| ²¹¹Bi | α | 6.67 | 2.17 minutes |
| ²⁰⁷Tl | β | 0.49 | 4.77 minutes |
| ²⁰⁷Pb | | | Stable |

**Table 2**

| Nuclide | Decay mode | Mean particle energy (MeV) | Half-life |
|---|---|---|---|
| ²²⁵Ac | α | 5.8 | 10 days |
| ²²¹ Fr | α | 6.3 | 4.8 minutes |
| ²¹⁷At | α | 7.0 | 32 ms |
| ²¹³Bi | β | 0.44 | 45.6 minutes |
| ²¹³Po | α | 8.3 | 4 µs |
| ²⁰⁹Pb | β | 0.6 | 3.25 hours |
| ²⁰⁹Bi | | | Stable |

As can be seen from the above tables, if an alpha emitter relatively high in the decay chain is used, there is the potential for a single nucleus to provide 4 or 5 highly energetic and highly cytotoxic alpha decays before reaching a stable isotope. This could potentially provide highly effective targeted cell killing. The problem with this, however, is that if the fate of these daughters is not controlled then for every one parent nucleus which decays at the desired site of action, 3 or 4 further alpha decays may take place at undesired sites. The power of such nuclei is thus very high but they demand exacting physical and chemical controls over their distribution.

Some methods involving the chelation of alpha-emitting metal nuclei have been proposed but these are generally inadequate for maintaining control over a whole decay chain starting at any radioisotope having one or more alpha-emitting daughter nuclei because simple chelation cannot maintain control over these daughter nuclei, for the reasons considered above. Certain methods have been proposed in the past to help control the distribution of daughter nuclei following alpha decay and so to allow effective targeting and use of alpha emitters in therapy. These methods include the incorporation of the alpha emitter into bone surfaces in need of treatment, where the daughters may remain trapped (e.g. WO 00/40275) and the incorporation of alpha emitters into liposomes such that the recoiling daughter nuclei remain trapped within the core region of the liposome (e.g. WO 01/60417).

A still further difficulty with the administration of alpha-emitting radionuclei is the destructive effect of the alpha radiation and the recoiling daughter nuclei on the composition. The very high energy of the decay event can not only break the radionuclide out of chelation but can have a considerably destructive effect upon the formulation itself. Thus, even if the radionuclide has a half-life of several days, it may be necessary to prepare a pharmaceutical composition directly prior to administration since the destructive energy released by decay of even a small amount of the radioisotope can disrupt the remainder of the composition.

One way in which the effectiveness of treatments for neoplastic disease, especially cancers, has been enhanced in recent years is by the concept of combination therapy. The idea behind combination therapy is that two or more drugs or methods for combating undesired cell populations are used simultaneously, or in relatively quick succession. In this way, target cells which may have been weakened by a first treatment method can be rendered more susceptible to killing by a subsequent method. In addition, the side effects of two or more treatment methods used in combination may be additive or preferably less than additive while the therapeutic effect is additive or preferably greater than additive. Thus, greater therapeutic effect can be provided using a treatment regime which remains tolerable to the patient. One of the most effective methods for providing combination therapy is to generate a medicament with more than one mode of activity. This results in the simultaneous treatment of target cells with more than one therapeutic agent. This may result in a greater than additive increase in effectiveness since, to survive, the cell must withstand two modes of attack simultaneously. Equally, any sub-population of target cells which might have some resistance to one mode of treatment will more likely be wiped out by the other mode and so the development of resistance will be retarded.

At present, there is no effective method for generating therapeutics which can control and deliver the high cytotoxicity of a heavy metal alpha emitting radionuclide and its daughter isotopes in combination with a secondary therapeutic (especially cytotoxic) agent in the same treatment delivery system. This is because to combine the two into the same molecule would rely upon a chelation effect to trap the alpha emitter, which would be inadequate to maintain control over the daughter nuclei. There is thus a considerable need for a medicament and delivery system which can provide a combination therapy including delivery and control over a heavy metal alpha-emitter in combination with the simultaneous delivery of a secondary agent in the same delivery system.

The difficulties faced in preparing an effective combination medicament including an alpha-emitting radionucleide are vastly greater than those faced in the preparation of, for example, combinations of beta-emitters and other therapeutics. Among these difficulties are the problems with maintaining control over the parent and daughter nucleii described above, the enormous and concentrated destructive force of the local alpha-irradiation, and the fact that long-lived isotopes cannot be administered because of the long-term damamge that such irradiation could cause to the subject. In the case of, for example, a beta-emitter, the nuclear recoil is relatively tiny, allowing a beta-emitter to be coordinated to another therapeutic agent by simple chelation. Similarly, the deposition of energy per unit volume from beta-emission is around four orders of magnitude less than for alpha-emission and thus any chemical structre in close proximity to alpha-irradiation must be capable of sustaining much greater damage, while remaining functional, than would be the case for beta-emitters. The preparation aspect is an additional significant difficulty. In particular, the efficient and robust control of alpha emitters must ideally be provided by a targeting agent which can be loaded quickly and easily, preferably at the "point of care" or very shortly before. Complex synthetic procedures cannot be undertaken in the short period available before the decay of a suitable alpha-emitter.

Combinations of alpha-emitters and other therapeutics have thus previously been seen as near-impossible to produce since they must have facile synthesis, robust control over the radionuclei and stability of the formulation to destruction or loss of the secondary medicament under prolonged high-energy bombardment

The present inventors have now, surprisingly established that liposomes can be prepared containing both an alpha-emitting heavy metal radionuclide and a secondary therapeutic agent being doxorubicin. More surprisingly still, they have established that such liposomes can be made sufficiently stable to the high energies of alpha decay and nuclear recoil that they can remain stable to storage for periods of several weeks and can stably retain the alpha emitter, the secondary therapeutic agent (doxorubicin) and in some cases the daughter nuclei after alpha decay. This is a considerable surprise since the liposome will be punctured many times by high-energy particles during this period.

In a first aspect, the present invention thus provides a cytotoxic agent comprising liposomes wherein said liposomes enclose a solution comprising at least one alpha emitting radionuclide as defined in the claims and at least one other therapeutic agent being doxorubicin. Preferably the alpha-emitting radionuclide is a heavy metal alpha-emitter, as defined in the claims. The liposomes preferably also enclose at least one chelating or complexing agent. The liposomes also preferably enclose at least one ionophore.

In a further aspect, the present invention also provides a method for the synthesis of liposomes enclosing a solution comprising at least one alpha emitting radionuclide as defined in the claims and at least one other therapeutic agent being doxorubicin, said method comprising contacting liposomes enclosing a solution comprising at least one therapeutic agent (doxorubicin) other than an alpha emitting radionuclide with a solution comprising at least one ionophore and at least one alpha emitting radionuclide as defined in the claims. Preferably, the method of the invention is a method for the formation of a cytotoxic agent of the invention.

In a further aspect, the present invention provides a pharmaceutical composition comprising at least one cytotoxic agent of the present invention and, optionally and preferably, at least one pharmaceutically tolerable carrier and/or excipient. In a still further aspect the invention provides a cytotoxic agent of the present invention for use in therapy.

The cytotoxic agents of the present invention and pharmaceutical compositions and formulations comprising such agents are highly suitable for use in the treatment of disease, particularly in the treatment of hyperplastic or neoplastic disease.

In a yet further aspect, the present invention thus provides a composition for use in the treatment of a disease, preferably a hyperplastic or neoplastic disease, said treatment comprising administering to a (human or non-human), preferably mammalian, subject a cytotoxic agent of the present invention. In a yet still further aspect, the present invention provides for the use of a cytotoxic agent of the present invention in the manufacture of a medicament for use in a method of treatment of hyperplastic or neoplastic disease. The cytotoxic agent should preferably be administered in a therapeutically, prophylactically and/or pain palliatingly effective amount.

The cytotoxic agents of the present invention comprise liposomes encapsulating a solution comprising at least one alpha-emitting radionuclide or at least one indirect alpha-emitting radionuclide as defined in the claims and at least one other therapeutic agent being doxorubicin. Preferably, the radionuclides of the present invention will be heavy metal radionuclides in that they will have a relative isotopic mass of at least 150 amu. Preferably, the radionuclides will have an isotopic mass of at least 200, more preferably between 210 and 230. Highly preferred heavy metal alpha-emitting radioisotopes include ²¹¹At, ²¹²Bi, ²²³Ra, ²²⁴Ra, ²²⁵Ac and ²²⁷Th.

The term "alpha-emitting radionuclide" as used herein encompasses nuclei with a single (alpha) mode of radioactive decay and also nuclei with multiple decay modes where at least a portion of the nuclei of this isotope decay by alpha-emission. Where a nucleus has more than one emission mode, it is preferably that at least 1% will decay by alpha-emission, preferably at least 10% and more preferably at least 30% will decay by alpha-emission. In one embodiment, substantially all nuclei will decay by alpha-emission. Where a "branching" alpha emitter having two or more decay modes produces a relatively low proportion of alpha particles, this may however also be an "indirect" alpha emitter, as described herein below, since the product of non-alpha decay may result (directly or indirectly) in a further alpha emitter. The alpha-emitters used in the present invention are: ²¹¹At, ²²³Ra, ²²⁴Ra, ²⁷⁵Ac, ²²⁷Th.

The term "alpha-emitting radionuclide" as used herein is also used to indicate, where context permits, an "indirect" alpha-emitting radionuclide. Such indirect alpha emitters may be radioisotopes which do not in themselves undergo alpha decay to a significant extent but decay by another mode (e.g. by beta-emission) to form an alpha emitter. Preferably this alpha emitter will be the direct daughter product of the "indirect" alpha emitter but may be the result of more than one non-alpha decay. Preferably the alpha-emitter formed from the decay of an "indirect" alpha emitter will have a short half-life (e.g. less than 24 hours, more typically less than 1 hour and preferably less than 10 minutes). The indirect alpha emitters of the present invention are ²¹²Pb, ²¹²Bi and ²¹³Bi (the latter two also being "branching" alpha emitters in themselves).

The half-life of the alpha-emitters will generally be sufficient to allow their preparation, transport and limited storage prior to use as a radiotherapeutic but will typically not be so long as to pose a long-term health risk to the subject if a certain amount of the isotope is retained in the body during and after treatment. Thus, the alpha emitters (including indirect alpha emitters) will typically have half-lives of at least 30 minutes, preferably at least 6 hours, and more preferably at least 1 day. Most preferred the used alpha emitters would have a half life of at least 3 days.

In order to avoid long term exposure, the half-life of the alpha emitters should generally be less than a year, preferably less than 6 months and more preferably less than 1 month. The body will also potentially be exposed to radiation from any alpha-emitting daughter generated by subsequent decay. Thus, where the decay chain from the administered alpha-emitter includes one or more other alpha-emitters, it is preferable that no isotope generated in that decay chain, up to and including the last alpha-emitting isotope before the formation of a stable nucleus, has a half life of greater than 1 year. More preferably this should be no greater than 6 months and most preferably no greater than 1 month.

The cytotoxic agents of the present invention typically comprise at least one alpha-emitting radionuclide or an indirect alpha-emitter as defined in the claims and at least one therapeutic agent being doxorubicin in combined amounts to achieve therapeutic, prophylactic and/or pain palliative effectiveness. This amount will evidently depend upon the particular isotope(s) selected for use, the nature and number of the other therapeutic agents, the condition(s) to be treated, prevented and/or palliated, the species, age, sex, weight, health and prognosis of the subject, the mode of administration, effectiveness of targeting, residence time, mode of clearance, type and severity of side effects of the pharmaceutical composition and upon many other factors which will be evident to those of skill in the art. Typically, the total radiation dose will be in the range of from 10 kiloBq to 10 gigaBq per each single administration, with a more preferred range being 1 megaBq to 1 gigaBq per each single administration. Similarly, doxorubicin and the other therapeutic agent(s) will be used at a level at which therapeutic, prophylactic and/or pain palliating effectiveness in combination with the alpha-emitter or indirect alpha-emitter of the present invention will be expected. Since the other therapeutic agent(s) will generally be known pharmaceutical agents they will typically be used at a level between 10% of their normal minimum therapeutic dose and 500% of their maximum normal therapeutic dose. More preferably this range will be 25% of the normal minimum dose to 200% of the normal maximum dose.

In one preferred embodiment, the total alpha emission level in the cytotoxic agents of the invention is below the minimum dose required for therapeutic, prophylactic and/or pain palliative effectiveness when used as a single therapy (e.g. 10-99%, preferably 25 to 75% of that minimum dose). This allows for reduction of the side effects caused by the endo-radionuclide therapy but the therapy is rendered effective because in combination with the at least one other therapeutic agent, the cytotoxic agents of the invention are effective overall. In a further preferred embodiment the, or each of the, other therapeutic agent(s) is used at a level below the minimum normal therapeutic dose, for example 10-99% of the normal minimum therapeutic dose, preferably 25 to 75% of their normal therapeutic dose. This again serves to reduce the danger of side effects and allows a higher level of total effectiveness without exposing the subject to unacceptable side effects.

In one preferred aspect of the present invention, the alpha-emitting radionuclide or indirect alpha-emitter of the present invention and the at least one other therapeutic agent being doxorubicin in the cytotoxic agents of the invention are synergistic with respect to their dosages. That is to say that the effect provided by the cytotoxic agent of the present invention is greater than would be anticipated from the additive effects of the incorporated doses of alpha-emitting radionuclide or indirect alpha-emitter of the present invention and at least one other therapeutic being dioxorubicin when used separately. In an alternative but equally preferred embodiment, the alpha-emitting radionudide or indirect alpha-emitter as defined in the claims and the at least one other therapeutic agent being dioxorubicin in the cytotoxic agents of the invention are synergistic with respect to their side effects. That is to say that the side-effects caused by the cytotoxic agent of the present invention are less than would be anticipated when the equivalent therapeutic effect is provided by either the alpha-emitting or indirect alpha-emitting radionuclide or by the at least one other therapeutic agent (dioxorubicin) when used separately.

A considerable advantage of one embodiment of the invention is that the cytotoxic agents are surprisingly able to maintain the daughter nuclei produced by alpha-decay of the radionuclide within the liposome. This is particularly important where the daughter nuclide and/or any other nuclide generated in the radioactive decay chain between the first radionuclide and a stable isotope is also an alpha-emitting radionuclide. Thus, in one embodiment, the liposomes preferably encapsulate a solution comprising and alpha-emitting radionuclide which generates at least one further alpha-emitting radionuclide during its decay. It is preferable that the liposome is capable of stably retaining this further alpha-emitting radionuclide. It is further preferable that the liposome is capable of retaining any subsequent alpha-emitting radionuclides which may be generated by further radioactive decay.

In alternative embodiments of the present invention, where the mother nuclei is an alpa emitter, as defined in the claims, the daughter nuclei may not be reatined to a significant extent. This is, however, acceptable under some circumstance, particularly where the decay of the daughter is rapid and thus the daughter isotope does not have time to significantly translocate after formation. Such rapid decay might be, for example, with a half-life of less than 1 hour, preferably less than 20 minutes and most preferably less than 1 minute. Similarly, where the half-life of the daughter is long relative to its rate of clearance from the body then little of the daughter will decay before it is expelled and so no significant harm will result. This might be with a half-life of at least 1 day, preferably at least 3 days and more preferably at least 10 days.

A liposome may be considered as capable of stably retaining the daughter nuclei generated from radioactive decay if at least 10% of such daughter nuclei resulting from that radioactive decay are retained within the solution entrapped by the liposome. Preferably at least 25% of the daughter nuclei are retained and more preferably at least 30% are retained within this encapsulated solution. This is a considerable advantage over other forms of alpha-radionuclide administration where effectively 100% of the daughter nuclei will be lost due to the nuclear recoil on decay. Where the cytotoxic agents of the present invention are formulated for non - systemic (especially local or regional) use, the liposomes may be larger and will retain a still greater proportion of the decaying alpha radionuclides. In these applications at least 40% and even at least 50% of the decaying alpha radionuclides may preferably be retained.

A surprising and advantageous aspect of the cytotoxic agents of the present invention is that they are capable of retaining the contents of the liposomes even under the high energy bombardment of local alpha-decay. In one embodiment, the cytotoxic agents thus retain at least 50% of the other therapeutic agent for greater than the half life of the alpha-emitting radionuclide. This is preferably greater than 60% and more preferably at least 75%.

The liposomes utilised in the present invention may be formed from any suitable lipid or mixture thereof and may be uni-lamellar, bi-lamellar or multi-lamellar. The present inventors have surprisingly established that liposomes are capable of maintaining their structure and retaining parent and daughter radionuclides and one or more additional therapeutic agents even under conditions in which they are exposed to the damage caused by a therapeutic level of alpha irradiation and the resulting nuclear recoil. Without being bound by theory, it is the inventors' belief that this surprising ability is the result of the liposomes' ability to "heal" when exposed to damage.

It is thus preferable that the lipid or lipid mixture forming the lamellar structure of the liposome should have a thermotropic transition temperature around or below physiological or room temperatures. In this way, the fluidity of the liposomal membrane is enhanced and its ability to "heal" is maximised. Consequently, in one embodiment of the invention, the liposomes are formed from a lipid or lipid mixture having a thermotropic transition temperature of around 10-50°C, preferably 18-45°C and more preferably 20-40°C. Where a mixture of lipids is utilised, not all components need have transition temperatures in these ranges but it is preferable that at least one lipid component should have such a transition temperature and more preferable that, as a whole, the mixture should have a transition temperature in this range.

In a preferred embodiment, the cytotoxic agents of the present invention are thus stable to the loss of their contents (especially the loss of the alpha radionuclide and/or the at least one other therapeutic agent) during storage for at least 3 day, preferably at least 1 week and most preferably at least 3 weeks when stored at room temperature. This stability is highly surprising since during this period of storage the liposomes of the cytotoxic agents will be repeatedly punctured by a level of alpha particles and recoiling daughter nuclei which will be at least equivalent to the therapeutic dose capable of extensive cell killing within the subject.

By stably retained in this context is indicated that no more than 20%, preferably no more than 10% and most preferably no more than 5% of the contents (especially the alpha radionuclide and/or the at least one other active agent) will be lost from the liposome into the surrounding solution during the storage period. Once again, without being bound by theory, the ability of liposome membrane to "self heal" after being "punctured" by alpha irradiation and/or recoil is believed to allow this surprising degree of retention.

As used herein the term "liposome" is used to indicate a vesicle structure comprising at least one lipid bilayer surrounding and enclosing at least one region of solvent. More than one bilayer may be present and so the liposome may be a uni-lamellar vesicle or a multi-lamellar vesicle (MLV). Similarly, more than one region may be enclosed by having solvent regions between the lamellae of a MLV and/or by two or more liposomes being fused or joined to enclose two or more regions of solvent separated by at least one lipid bilayer. Any solvent region essentially surrounded by one or more lipid bilayers is considered to be an enclosed region. Where more than one solvent region is enclosed, these will generally comprise substantially similar solvent and solution components.

The solvent region enclosed by the liposomes of the present invention will generally be an aqueous solution having dissolved or suspended therein at least one alpha-emitting or indirect alpha-emitting radionuclide as described herein and at least one other therapeutic agent being doxorubicin. The solvent region may additionally comprise other pharmaceutically tolerable components, especially including chelating and/or complexing agent and/or ionophores, pH modifiers, tonicity modifiers, targeting agents .

Liposomes without targeting groups tend to be passively incorporated in solid tumours due to tumour-associated vascular fenestration. This allows for "passive targeting of the dual/multiply active cytotoxic agents of the present invention where the disease to be treated includes a solid tumour. The mechanism responsible for tumour uptake of liposomes is likely to be the capillary leakage often found in neovasculature of tumours allowing liposomes to diffuse through capillary walls and into the interstitium of tumours but not normal tissues.

The liposomes of the present invention may be modified in order to increase their ability to target the desired site of action of the alpha-emitter or indirect alpha-emitter and/or the at least one other therapeutic agent (doxorubicin) and/or may be modified in order to control their clearance rate and/or mode of clearance. In particular, the rate of clearance of liposomes *in vivo* may often be reduced by surface modification of the liposome with a polyalkylene glycol containing molecule. Such molecules will generally have at least one polyalkylene chain (e.g. polyethylene, polypropylene or mixtures thereof) and at least one membrane-affinic group (e.g. a fatty acid chain or C₈ to C₂₄ alkanyl or alkenyl group). Examples include polyethylene glycol grafted lipids and/or a polypropylene glycol modified membrane-soluble molecules. Such surface modification will be familiar to one of skill in the art. Surface modified liposomes are highly preferred, particularly for systemic use, because they generally have a longer residence time in the blood stream. As a result they have a greater opportunity to take advantage of the "leaky" nature of the tumour (neo)vascilature and so may provide a greater tumour targeting effect.

Liposomes may be effectively targeted to their desired site of action by surface modification with at least one targeting moiety. Suitable targeting moieties include polypeptides such as cell surface receptors, receptor fragments, cell adhesion molecules and fragments thereof, antibodies, antibody constructs, antibody fragments and single chain antibodies, hormones or hormone analogues such as oestrogens and testosterones and small molecules such as folates or folate analogues. In particular, targeting moieties may advantageously be moieties which bind to cell surface or extracellular agents produced in unusually high concentration by target cells. Such agents include hormone receptors such as testosterone receptors and/or oestrogen receptors. By conjugating monoclonal antibodies or other molecules with affinity for tumour cell associated receptors to liposomes, they can actively target single cell or micrometastatic disease, as well as even more effectively localise to solid tumours.

In one embodiment, the liposomes are not surface modified with folate conjugated to a monoclonal antibody or FAB fragment thereof.

The liposomes used in the present invention will typically be suitable for systemic, regional and/or local therapy. Liposomes for local or regional (e.g. intracavitary) use will typically be larger than those intended for systemic application to the blood stream. Liposomes suitable for administration to the blood stream (e.g. by intravenous or intraarterial injection or infusion) will generally be no larger than 10µm in their largest dimension, preferably no larger than 1 µm and more preferably no larger than 200 nm. Since very small liposomes may not retain their contents so efficiently under the considerable destructive effect of therapeutic alpha-irradiation (and resulting nuclear recoil), the liposomes should generally be no smaller than 10 nm in maximum dimension, preferably no smaller than 20 nm and more preferably no smaller than 40 nm. In one preferred embodiment, the liposomes used in the cytotoxic agents of the present invention are larger than 100 nm (e.g. 105 nm or larger) in their maximum dimension. For treatment other than by administration to the blood stream, the maximum dimension of the liposomes will typically be larger and may be up to 100 µm, preferably up to 50 µm and more preferably up to 30 µm. In order to provide low clearance in these administration methods, the liposomes may be at least 100 nm in maximum dimension, preferably at least 2 µm and more preferably at least 5 µm.

In the cytotoxic agents of the invention, the sample of agent as a whole will include liposomes which contain both an alpha-emitting or indirect alpha-emitting radionuclide as defined in the claims and at least one other therapeutic agent being doxorubicin. Generally, the level of alpha radionuclide necessary to have a therapeutic effect will be very low in comparison with the level of other therapeutic agent(s) because of the extremely high cytotoxicity of alpha radiation. As a result, although a large proportion of the liposomes will contain the other therapeutic agent (doxorubicin), not all need contain alpha radionuclide.

It is desirable that at least 80% by volume of the liposomes contain at least one therapeutic agent other than an alpha-emitting radionuclide. This will preferably be at least 90% and will most preferably be at least 95% (e.g. substantially 100%). It is also desirable that at least 10% by volume of the liposomes contain both the radionuclide and the other active agent. This proportion is preferably at least 20%, more preferably at least 30% and most preferably at least 45%.

In a further embodiment, the cytotoxic agents of the invention may comprise liposomes containing both at least one alpha-emitting or indirect alpha-emitting radionuclide as defined in the claims and at least one other therapeutic agent being doxorubicin as indicated herein and also contain a small proportion (30% or less by volume) of additional liposomes containing only the other therapeutic agent (doxorubicin) or preferably containing only radionuclide. This allows the properties of the cytotoxic agent to be "fine tuned" for a particular application prior to administration. Such additional liposomes are preferably mixed in shortly before administration.

Suitable liposomes for use in forming the cytotoxic agents of the present invention may be formed by methods known in the art or may be purchased commercially with appropriate compositions, sizes and optionally surface modified. In one preferred embodiment, the cytotoxic agents are formed from commercially available liposomes pre-loaded with at least one non-radioactive therapeutic agent. Pegylated liposomal formulations of doxorubicin are particularly preferred and may be synthesised or sourced comercially. A highly preferred pre-loaded liposome is a liposomal formulation of doxorubicin, such as the commercial formulation Caelyx (RTM).

The "other therapeutic agent" other than doxorubicin referred to herein is an agent suitable for use in therapy in combination with an alpha-emitting radionuclide or indirect alpha-emitting as described herein. Such therapeutic agents other than doxorubicin may be present in order to reduce undesirable side-effects of the alpha-emitting radioisotope but will typically be agents which are in themselves capable of killing or limiting the growth of at least a portion of the target cells. In particular, since both the alpha emitting radioisotope and the other therapeutic agent(s) are distributed with the liposomes, it is preferable that all are cytotoxic and highly preferable that they are cytotoxic by means of different mechanisms. This allows the greatest opportunity for gaining an advantage from combination therapy since cells which are resistant to one mechanism may be susceptible to another and the stress caused to the target cell by one agent may leave it weakened in respect of the other agent. Suitable other therapeutic agents other than doxorubicin include radioisotopes emitting other forms of radiation, such as beta particles, gamma rays, X-rays, conversion electrons, Auger electrons or combinations thereof; chemotherapeutic agents such as folate analogues, nucleoside analogues, toxins, angeogenesis inhibitors etc; nucleic acid based therapeutics such as genes operably linked to promoters, antisense DNAs and/or RNAs and small interfering DNAs/RNAs; viruses, either active or inactivated, as such, or as carriers for nucleic acid therapeutics; immunotherapeutics including optionally functionalised monoclonal antibodies, constructs or fragments thereof; and agents for enhancing other therapies such as boron for use in boron capture therapy.

Other therapeutics also include imaging and image enhancing agents in order to facilitate visualisation of the target site and/or the distribution of the cytotoxic agent. Such imaging agents include gamma and/or X-ray emitting isotopes, magnetic resonance contrast agents (e.g. gadolinium complexes), ultrasound contrast agents and/or X-ray contrast enhancing agents (e.g. heavy metal or iodine containing compounds).

Preferably, where the other therapeutic agent is an imaging agent, this therapeutic agent will also have cytotoxic effect (such as a cytotoxic beta-emitter with a measurabel gamma-emission) or will be used as a third or further therapeutic agent in combination with the alpha emitter and at least one cytotoxic "other" therapeutic agent.

It is preferable that the other therapeutic agent is not an alpha emitter. Where the other therapeutic agent is a radionuclide with more than one decay mode it is preferable that less than 1%, preferably less than 0.5% decay by alpha-emission.

In the present invention doxorubicin is encapsuled in the liposomes.

The cytotoxic agents of the present invention preferably encapsulate a solution comprising at least one complexing/chelating agent. By providing a suitable concentration of at least one appropriate complexing/chelating agent the liposomes may retain the parent and daughter radionuclides to a greater extent and thus exert more effective control over the *in vivo* distribution of the cytotoxic effect. The solution within the liposomes may also contain other agents to reduce the chance of nuclear recoil propelling the daughter nucleus out of the liposome or the radioactive decay causing the radionuclide and/or any other therapeutic agent being doxorubicin to escape through any transient holes in the lipid membrane. Suitable other internal agents include water soluble polymers or other viscosity modifying agents (to slow diffusion of the active agents out of the liposome in the event that a transient hole is made) and large cross-section ions, such as (radioactive or preferably non-radioactive) heavy metal ions to reduce the distance a recoiling daughter nucleus will travel before a collision.

The complexing/chelating agent(s) in the solution enclosed by the liposomes will typically be at least one poly-dentate macrocyclic chelating agent such as cyclic poly-ethers and/or substituted or unsubstituted cyclic polyamines, cyclic polythioethers, or cyclic compounds having and mixture of heteroatoms. Suitable ring sizes for the chelants, which may be mono-cyclic or polycyclic are preferably around 8 to around 20 atoms, preferably around 10 to around 18, with between 2 and 10, preferably between 3 and 7 heteroatoms in the ring. The cyclic chelant(s) may also be substituted at any carbon or suitable heteroatom (such nitrogen) with optional substituants. These optional substituants may include one or more further complexing groups such as carboxylic acid, phosphonic acid, amide, amine (primary, secondary, tertiary or quaternary), ester or ether groups and/or may serve other purposes such as linking the chelants into or onto a polymer backbone so as to increase the local concentration or chelating moieties. Some examples of suitable chelating agents include:
1,4,7,10 tetraazacyclododecane-1,4,7,10 N,N',N",N"'-tetraacetic acid (DOTA);
1,4,7,10 tetraazacyclotridecane-1,4,7,10 N,N,N",N"'-tetraacetic acid (TRITA);
1,4,7,10 tetraazacyclotetradecane-1,4,7,10 N,N',N",N"'-tetraacetic acid (TETA);
1,4,7,10 tetraazacyclododecane-1,4,7,10 N,N',N",N"'-tetra(methylene) phosphonic acid (DOTMP);
1,4,7,10 tetraazacyclotridecane-1,4,7,10 N,N,N",N"'-tetra(methylene) phosphonic acid;
1,4,7,10 tetraazacyclotetradecane-1,4,7,10 N,N',N",N"'-tetra(methylene) phosphonic acid;
diethylene triamine N,N',N" pentaacetic acid and isomeric derivatives thereof;
cryptate[2,2,2], cryptate[3,2,2], cryptate[2,2,1] and mono and di-benzo derivatives thereof;
bridged calix[4]arenes containing electron rich groups selected from hydroxyl, carboxyl, ester, amide, and amine;
1,10 diaza-4,7,13,16-tetraoxacyclooctadecane 1,10 N,N' bis-acetic acid; and
1,10 diaza-4,7,13,16-tetraoxacyclooctadecane 1,10 N,N' bis-malonate.

The chelators will preferably be selected to correspond to the common valances and oxidation states of the heavy metal radionuclide(s) and, if appropriate, any daughter radionuclides which are desirably retained within the liposome. For example, if the initial alpha-emitting radionuclide comprises ²²⁷Th, then this is a transition metal most stable in the 4+ oxidation state but, on alpha decay, generates ²²³Ra which is an alkaline earth metal adopting a 2+ oxidation state. Since ²²³Ra is also an alpha emitting radionuclide, it is important to retain as large a proportion of both the parent and daughter isotopes as possible. Suitable liposomes may therefore contain chelation agents suitable for retaining both 4+ transition metals and 2+ alkaline earth metals, as well as optionally chelants suitable for retaining the ions of other nuclides in the decay chain (which chelants may, of course be the same as those previously considered for the first two ions). Evidently, where the half-life of an isotope is very short, however, it will be less important that corresponding chelation is present.

In one embodiment, the cytotoxic agents of the invention thus comprise liposomes which encapsulate a solution comprising at least one alpha emitting radionuclide or indirect alpha-emitting radionuclide, and doxorubicin as defined in the claims as well as chelants suitable for chelating to one or more common ion of each radionuclide in the decay chain beginning with said alpha emitting radionuclide and ending with the last alpha emitting radionuclide before a stable isotope is reached. Chelation need not be provided, however, for very short lived nuclei and thus these chelants may be with the exception of chelation for any isotope having a half-life of less than 10 seconds, preferably less than 5 seconds and most preferably less than 1 second. Chelation may also not be provided where a radionuclide is an unreactive element, such as a noble gas, although in such cases viscosity effects may preferably be used to reduce the rate of diffusion, as also may elevated concentrations of heavy metal ions.

Since ionophores, as described herein, also serve to chelate the heavy atom ions used in the present invention (as described herein below), the "chelators" as described herein may, in one embodiment of the invention be ionophores. Where this is the case, the ionophore and chelant components of the cytotoxic agents of the invention may be the same compound and will be present at a level sufficient to be effective in both roles.

In an alternative embodiment, the chelant may be present in addition to any chelating effect provided by the ionophore. In such an embodiment, the chelant referred to herein will not be the ionophore component.

Furthermore, since certain bioactive agents may serve to complex the alpha emitting radionuclides and/or daughter radionuclides of the present invention, the complexing agent may also be an "other therapeutic agent" as described herein. Doxorubicin is also a chelating therapeutic agent.

The cytotoxic agents of the present invention also preferably encapsulate a solution comprising at least one ionophore. Suitable ionophores allow transport of the alpha-emitting radionuclide across the (at least one) membrane bilayer of the liposome and also server to help capture the (especially parent) radionuclide within the liposome. Suitable ionophores include any molecule capable of transporting a heavy metal ion across a membrane bilayer. Some ionophores (such as calcium ionophore A 23187) occur naturally and may be used in the form of a largely or highly purified extract and others can be formed synthetically. Synthetic ionophores preferably include two or three substituted amide groups for chelation of the metal ion and several (especially two per amide group) hydrophobic substituents to provide sufficient lipid solubility. Suitable groups being, for example, C₁ to C₁₈, especially C₃ to C₁₀, alkyl substituents.

Calcium and magnesium ionophores such as A 23187; N,N,N',N'-tetrabutyl-3,6-dioxaoctanedi[thioamide]); ; N,N,N',N'-tetracyclohexyl-3-oxapentanediamide; N,N-dicyclohexyl-N',N'-dioctadecyl-diglycolicdiamide; N,N'-diheptyl-N,N'-dimethyl-1,-butanediamide; N,N"-octamethylene-bis[N'-heptyl-N'-methylmalonamide], and others well known in the art may be particularly suitable for metal ions having a stable 2+ oxidation state, especially Ra²⁺ such as ²²³Ra and/or ²²⁴Ra. Similarly, known Pb ionophores such as N,N-dioctadecyl-N',N'-dipropyl-3,6-dioxaoctanediamide may be particularly suitable for heavy metal ions in the 4+ oxidation state such as Th⁴⁺ (e.g. ²²⁷Th).

The cytotoxic agents of the present invention are highly suited for specific cell killing and thus are effective for use in the treatment of hyperplastic and/or neoplastic diseases and for the manufacture of medicaments for such treatment. The cytotoxic agents of the invention are particularly suited for use in eliminating diseased cells in benign, malignant and/or metastatic cancers and/or leukaemias.

Specific examples of diseases suitable for treatment by the agents of the present invention include small cell and non-small cell lung cancer, malignant melanoma, ovarian cancer, breast cancer, bone cancer, colon cancer, bladder cancer, cervical cancer, sarcomas, lymphomas, leukaemias and tumours of the prostate. Other diseases particularly applicable to the application of the invention include non-cancerous, especially hyperplastic diseases and for the reduction of pain in diseases (especially diseases pf the bone) including arthritis. Furthermore, the cytotoxic agents of the invention can be used to target the reticuloendothelial system (RES) and thus can be used for immunomodulation. Preferred diseases for such treatment include autoimmune and tissue rejection diseases and in particular the agents of the invention may be used to help prevent graft rejection in transplantation surgery.

Thus, one embodiment of these aspects relates to a composition for use in the treatment of a disease and especially a tumour locally. Such treatment may preferably be applied through an intratumoral injection or infusion to a subject (generally a human patient) in need of such treatment, of a therapeutically effective amount of a cytotoxic agent according to the invention. Such a method provides local irradiation of the tumour tissue and/or tumour neovascilature and also delivery of at least one other therapeutic agent in combination therewith. Alpha emitting radionuclides as defined in the claims are highly effective in this embodiment because their range is short and damage to surrounding healthy tissue is minimised. Examples of diseases which may benefit particularly from this embodiment of the invention are those causing solid tumours, such as non-small cell lung cancer, malignant melanoma, ovarian cancer, colon cancer, sarcomas, and tumours of the prostate.
A further embodiment of the invention relates to a compositions for use in the treatment of locally disseminated cancers, such as for instance liver tumours, or peritoneally or intracranially confined diseases. This treatment may especially preferably be applied through loco-regional injections or infusions, to a subject in need of such treatment, of a therapeutically effective amount of a cytotoxic agent or composition according to the invention.

Still another embodiment of the invention is a composition for use in the treatment of locally disseminated cancers such as liver tumours, through administration of a therapeutically effective amount of a liquid preparation comprising a cytotoxic agent of the invention to a subject in need of such treatment, especially to said subject's blood supply to the affected area or organ, e.g. to the blood supply to the liver in the case of a liver tumour. This may promote transport of the agent/composition into the tumour.

A further embodiment of the invention relates to a composition for use in the treatment of systemically disseminated cancer by intravenous injection or infusion, or other systemic administration, to a subject in need of such treatment, of a therapeutically effective amount of a pharmaceutical preparation comprising a cytotoxic agent according to the invention.

A further embodiment of the invention relates to a composition for use in the treatment of intracavitary tumours, where a therapeutically effective amount of a cytotoxic agent of the present invention is administered to a subject in need of such treatment by, for example, injection or infusion into the tumour affected cavity and retained there for a sufficient period to obtain a therapeutic effect on the cavity surfaces. Such cavities include the cranial cavity, peritoneal cavity and cavities created by pericardial effusion and mesothelioma and administration is suitable for cancers such as intracranial cancers, intraperitoneal cancers or cancers located in the cavities created by pericardial effusion and mesothelioma.

In a further embodiment, the present invention provides for the use of a cytotoxic agent of the invention as a local, regional and/or systemic follow-up treatment following other methods of therapy, particularly external beam irradiation and particularly surgery. In this embodiment the cytotoxic agent may be administered in order to help eliminate any remaining diseased cells which were not killed by other methods or were not removed by surgery. In particular, where a solid tumour is resected from a body cavity, the site of attachment of the tumour and/or the surface(s) of the cavity may be treated in order to reduce the risk of diseased cells remaining. This would be a local treatment embodiment, where the cytotoxic agent was formulated as a liquid, gel, cream, ointment, paint, spray or similar for direct application during the surgical procedure. A gel, cream, ointment or paint, especially a gel is highly preferred in this embodiment. Alternatively (or in addition), the cytotoxic agents may be administered to the cavity after surgery, for example by intra-cavity injection or infusion to achieve a similar, post-surgery sterilisation, effect.

In the case of known, suspected or otherwise indicated metastatic disease, the agents of the present invention may be applied systemically in combination with surgical resection or other treatment of the primary tumour. In such cases the agents of the invention will generally be formulated as injectable and/or infusible liquids suitable for administration to the blood stream.

As indicated above, in appropriate applications the agents of the present invention may be formulated as pharmaceutical compositions in the form of injectable and/or infusible liquids (optionally for dilution before administration), as creams, gels, soluble or insoluble patches, pastes, paints, ointments, sprays, impregnated absorbable or non-absorbable gauzes and other suitable formulations known to one of ordinary skill in the art. Such compositions will desirably comprise at least one pharmaceutically tolerable carrier or excipient such as water for injection, sterile saline; buffers, thickeners, colorants, stabilisers, pH adjusters, viscosity modifiers, tonicity modifiers, salts, sugars, physical supports and other agents well known to the skilled worker. The compositions may be administered by any suitable method, such as infusion by means of a catheter, injection by means of a standard needle and syringe arrangement or a needleless syringe, or direct application of a local formulation, such as by spraying, painting etc.

In a preferred embodiment of the invention, the cytotoxic agents described herein are administered as part of a treatment regime comprising at least three-stages:
a) Administration of liposomes comprising at least one imaging or image enhancing agent whereby to assess the liposome distribution and potential tumour targeting;
b) Pre-treatment with at least one dose of non-radioactive liposomes whereby to reduce accumulation in the liver/ reticuloendothelial system (RES).
c) At least one administration of a cytotoxic agent of the present invention.

Generally, steps a), b) and c) will be carried out in that order, although the liposomes of step a) may also serve as the pre-treatment of step b), in which case these steps are accomplished simultaneously and the pre-treatment of step b) may be conducted before imaging step a)... A period of 12 hours to 10 days, preferably 24 hours to 7 days, more preferably 2 to 6 days is preferably allowed between pre-treatment step b) and administration step c). The liposomes of steps a), and c) will preferably comprise essentially the same lipid or lipid mixture in essentially the same proportions and will have essentially the same average size and size distribution such that the distribution of liposomes in step a) accurately reflects the therapeutic distribution in step c). In a highly preferred embodiment the liposomes of step b) will carry at least one therapeutic agent other than an alpha-emitting radionuclide. Particularly in this embodiment, it is preferably that the liposomes of step b) also have similar characteristics to those of steps a) and c), as discussed above.

The above method may be enhanced by one or more repetitions of the complete method and/or of steps b) and c).

In an alternative to the above method, for example where visulisation by use of liposomes is not required, steps b) and c) of the above method may be used without or independently of step a). Steps b) and c) provide a highly preferred administration method for the cytotoxic agents of the present invention since uptake to certain non-target tissues such as the liver can be controlled in this way. A preferred method is to administer liposomes containing the "other" therapeutic agent but no alpha-emitter in step b) and then administer the corresponding cytotoxic agent in step c) at a time period after as described above.

The present invention provides a method for the synthesis of liposomes enclosing a solution comprising at least one alpha emitting or indirect alpha-emitter as defined in the claim radionuclide and at least one other therapeutic agent being doxorubicin. This method comprises contacting liposomes enclosing a solution comprising at least one therapeutic agent (doxorubicin) other than an alpha emitting radionuclide with a solution comprising at least one ionophore and at least one alpha emitting radionuclide or indirect alpha-emitter as defined in the claims. Preferably, the method of the invention is a method for the formation of a cytotoxic agent of the invention as described herein. The liposomes are preferably as described herein above and will preferably contain at least one chelating agent in addition to the at least one other therapeutic agent.

In an alternative method of the invention, the other therapeutic agent being doxorubicin may be loaded into the liposome after and/or simultaneously with the loading of the liposomes with the radionuclide. Where the loading is simultaneous, this may be during liposome formation/reformation or may be after the liposomes have been generated.

The method of the present invention may comprise contacting liposomes enclosing a solution comprising at least one therapeutic agent (doxorubicin) other than an alpha emitting radionuclide and at least one chelant (which may be an ionophore as described above) with a "loading solution" comprising at least one ionophore and at least one alpha emitting radionuclide or indirect alpha-emitter as defined in the claims. Alternatively, the total concentration of chelant my be provided by transport across the liposome membrane from the loading solution.

The contact of liposomes with the loading solution is preferably carried out at a temperature above physiological temperature. This temperature is thus preferably around 45-90°C, more preferably around 50-80°C and most preferably around 60-75°C. Furthermore, the ionophore may preferably be selected such that it is capable of transporting the heavy metal radionuclide across the liposome membrane at elevated temperature (as described) but not at physiological and/or room and/or refrigerated storage temperature. Selection of a suitable ionophore for this aspect will depend upon the lipid(s) used in the liposomes and may be achieved by routine testing of loading effectiveness in the method of the invention and loading stability at physiological/room/refrigerated storage temperatures. This will be easily accomplished by one of normal skill in the art, who will appreciate that the number and nature of the hydrophobic groups in the ionophore will relate to the ease with which the molecule can cross the lipid membrane.

The method of the present invention may additionally comprise the step of forming liposomes containing said at least one therapeutic agent (doxorubicin) other than an alpha-emitting radioisotope prior to contact with the loading solution. Such methods are well known in the art and may be used to provide any desired combination of active agents and/or chelants and/or other agents as indicated herein. Alternatively, liposomes containing certain therapeutic agents are commercially available in pharmaceutical purity and may be used as the starting material in this respect.

During or after the formation of the liposomes and/or after their contact with the loading solution, the liposomes may be surface functionalised and/or surface modified to provide desirable residence and/or targeting characteristics as described herein.

It is preferred that all formation and fuctionalisation steps for the liposomes be carried out prior to their loading with alpha-emitting radionuclide. In this way, the liposomes may be produced efficiently on a relatively large scale and stored, if necessary for periods greater than several half-lives of the alpha-emitting radionuclide. These pre-prepared liposomes (which form a preferred element of a kit of the invention as described herein) may then be loaded with radionuclide relatively shortly before administration. In this way, a laboratory may maintain a stock of the liposome component and source the radionuclide as required for simple loading by the method of the invention. This provides a considerable advantage in allowing the routine use of relatively short lived alpha-radionuclides in combination therapy without difficulty and extensive preparation steps each time a batch is required. In this embodiment of the method of the invention, an ionophore is contacted with a fresh sample of a suitable alpha-emitting radionuclide (as described herein) whereby to form a solution of said ionophore and said alpha radionuclide. This solution is then contacted with suitable liposomes according to the method of the invention as described herein.

Also disclosed is a kit for the formation of a cytotoxic agent comprising liposomes wherein said liposomes enclose a solution comprising at least one alpha emitting radionuclide or indirect alpha-emitter as defined in the claims and at least one other therapeutic agent being doxorubicin, said kit comprising;
liposomes encapsulating a solution comprising said at least one other therapeutic agent (doxorubicin) and preferably at least one chelating agent, said liposomes preferably being in the form of an aqueous suspension;
said at least one alpha emitting radionuclide or indirect alpha-emitter as defined in the claims;
an ionophore, preferably in the form of a solution.

Optionally, the kits described in the context of the present invention also comprise other pharmaceutically acceptable carriers or excipients and/or means for the administration of the cytotoxic agent and/or instructions for the formation of cytotoxic agents of the present invention.

Since the cytotoxic agents of the invention may be generated simply from suitable pre-prepared liposomes by the method of the invention, in a further aspect it is herein described a kit comprising
liposomes, optionally and preferably pre-loaded with a solution comprising at least one therapeutic agent being doxorubicin other than an alpha emitting radionuclide and preferably at,least one chelating agent, said liposomes preferably being in the form of an aqueous suspension;
an ionophore, preferably in the form of a solution; and
optionally and preferably instructions for loading said liposomes whereby to generate a cytotoxic agent of the present invention, said instructions most preferably indicating that the ionophore should be contacted with a fresh sample of alpha radionuclide or indirect alpha emitter as defined in the claims whereby to form a solution of said ionophore and said alpha radionuclide or indirect alpha-emitter and subsequently or simultaneously the thus generated solution contacted with said liposomes.

### Examples

### MATERIALS AND METHODS

### Preparation of radionuclide

Radium-223 was produced from ²²⁷Ac (t1/2 = 21 y) and 227Th (t1/2 = 18.7 d) according to the previously presented method of Henriksen (Henriksen et al. Radiochim Acta 89, 661-666 (2001)). Briefly, ²²³Ra was separated from the actinides ²²⁷Ac and ²²⁷Th by the use of Ac-resin followed by cation exchange chromatography and filtration through a sterile filter (Millex GV 0.22 mm, Millipore Carrigtwohill Co, Irland).

### Example 1 - Loading of ²²³Ra into liposomes

1.1 Liposomal doxorubicin (Caelyx®, Schering Plough AS, Eiksmarka, Norway) corresponding to 2 mg doxorubicin per ml was subject to buffer exchange with a buffer containing 20 mM HEPES and 300 mM sucrose adjusted with NaOH to pH 7-8, and concentrated 3 times by a centrifuge concentration cartridge (Millipore UFV2BTK10, 30 KNMWL membrane, 15 ml max volume, Millipore, Bedford, IL, USA) inserted into an Eppendorf Centrifuge 5810R (Eppendorf, Germany) operated at 20 OC and 1400 rcf. Calcium inophore (Ca-ionophore, Calcimycin, Sigma, St Louis, MI, USA) was dissolved to a concentration of 1 mg per ml in chloroform. Approximately 15 ml was added to 2 ml vial and the chloroform evaporated of in a stream of argon to generate a thin film of Ca-ionophore on the inner surface of the vial. The ²²³Ra solution was diluted in a solution of sucrose (300mM) and HEPES (20 mM).

The vial was preheated to 60 oC and 200 ml of concentrated Caelyx was added. The loading mixture was shaken for 45 minutes on a thermomixer (Eppendorf, Germany) followed by the addition of 200 ml 10 mM EDTA solution. After 5 -10 minutes further shaking, the mixture was transferred to a Sephadex G-25 PD-10 column and eluted with 0.9% NaCl solution. The fraction containing liposome had a visible red colour and was collected and added 10% of a 10 ' Dulbecco's Modified Eagles,s Medium (Sigma, MI, USA). Subsequently, inside a sterile hood, the liposomes were sterile-filtered through a 0.22 µm sterile filter (Millex GV 0.22 µm, Millipore Carrigtwohill Co, Irland) into a 10 ml vial that was subsequently capped with a metal/rubber cap.

The vial was stored for at least three hours to reach equilibrium between ²²³Ra and the daughter nuclides before quantification of radioactivity was performed using a Capintec dose calibrator, which was calibrated for the ²²³Ra series in equilibrium with daughter nuclides.

### 1.2 Loading yield

The amount of ²²³Ra loaded into the Caelyx® liposomes was in the order of 51 to 67 % for three individual experiments. A control experiment using ²²³Ra and liposomes under identical conditions, except for the absence of ionophore, showed less than 1% of the ²²³Ra in the liposome fraction eluted from the Sephadex G-25 column.

### Example 2 - Animal study

10 nude mice were prepared with OHS osteosarcoma xenografts. Each mouse was trated subcutaneously with one dose of 375 kBq/kg of 223Ra encapsulated in Caelyx lipsomes as prepared in Example 1.

After 1 hour, one day and four days, three, three and four animals were sacrificed respectively and and the tissue distribution of radioactive material was measured.

The distribution of radionuclide was also compared with a control in which the radium was administered as a salt in a solution of liposomes but not encapsulated thereby.

### Results:

The data indicate that the radioactivity blood clearance is consistent with what was the expected clearance for this type of liposomes. Liver uptake appeared to be relatively low for the whole period studied while there was a significant uptake in the spleen. The uptake into the skeleton, as reflected by the activity in femur and skull, was increasing with time, probably because of a slowly catabolism of liposomes causing release of ²²³Ra.

There was a distinct difference between the distribution over time for the two ²²³Ra forms. This difference was particularly high in the blood, partly because of the slow clearance of the liposomes from blood and partly due to the rapid clearance of free ²²³Ra. For most soft tissues, the retention of the liposomal form was significantly elevated.

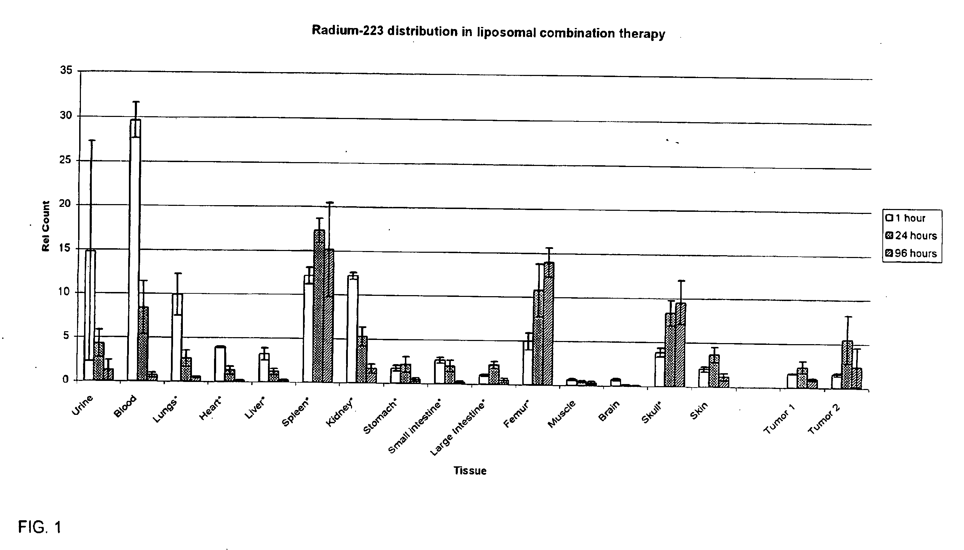

The tumor uptake of the liposomal form was initially modest due to the low vascularization in this tumor model, but incrased with time. The retention in tumor was higher than in most of the other soft tissues (Table 3 and Figure 1).

Conclusion: ²²³Ra administered as a liposomal combination therapy with doxorubicin had a relevant biodistribution in nude mice.

**Table 3**

| **Sample** | **Time Pont 1** | | **Time Point 2** | | **Time Point 3** | |
|---|---|---|---|---|---|---|
| | **1 hour** | **Stdev** | **24 hours** | **Stdev** | **96 hours** | **Stdev** |
| Urine | 14.79 | 12.53 | 4.30 | 1.60 | 1.25 | 1.17 |
| Blood | 29.65 | 1.98 | 8.36 | 3.00 | 0.72 | 0.27 |
| Lungs* | 9.86 | 2.35 | 2.62 | 0.95 | 0.51 | 0.09 |
| Heart* | 3.95 | 0.09 | 1.28 | 0.47 | 0.15 | 0.07 |
| Liver* | 3.18 | 0.70 | 1.18 | 0.33 | 0.24 | 0.07 |
| Spleen* | 12.11 | 0.93 | 17.31 | 1.37 | 15.13 | 5.37 |
| Kidney* | 12.12 | 0.38 | 5.29 | 1.06 | 1.64 | 0.52 |
| Stomach* | 1.72 | 0.32 | 2.14 | 0.90 | 0.49 | 0.18 |
| Small intestine* | 2.66 | 0.33 | 2.01 | 0.65 | 0.23 | 0.13 |
| Large Intestine* | 1.02 | 0.09 | 2.19 | 0.40 | 0.47 | 0.24 |
| Femur* | 4.96 | 0.96 | 10.74 | 3.01 | 13.93 | 1.68 |
| Muscle | 0.64 | 0.10 | 0.45 | 0.10 | 0.30 | 0.17 |
| Brain | 0.69 | 0.11 | 0.11 | 0.08 | 0.05 | 0.07 |
| Skull* | 3.86 | 0.49 | 8.32 | 1.43 | 9.51 | 2.46 |
| Skin | 1.98 | 0.26 | 3.65 | 0.87 | 1.13 | 0.40 |
| | | | | | | |
| Tumor 1 | 1.54 | 0.04 | 2.31 | 0.69 | 0.91 | 0.14 |
| Tumor 2 | 1.51 | 0.17 | 5.49 | 2.73 | 2.36 | 2.20 |

### Example 3 Biodistribution and Clearance with Pretreatment

For Examples 3 and 4, Normal Balb/C mice of both sexes (around 12 weeks and weighing 18-25g) were used, as were loaded liposomes produced in accordance with Example 1. The research protocol was in accordance with the European Convention for the Protection of Vertebrate Animals used for Experimental and other Scientific Purposes (ETS 123) issued by the Council of Europe and approved by The Norwegian Animal Research Authority. The mice were housed under standard condition and had access to food and water ad libitum.

### Optimisation of pretreatment

Before the injection of liposomal ²²³Ra, the mice were pre-treated with doxorubicin liposomes (Caelyx®) to reduce the reticulo-endothelial-system (RES) uptake of the product. An optimisation study was conducted ahead of the main blood clearance and biodistribution experiment to determine a favourable time interval between the pre-treatment and the main treatment. In the optimisation study the mice were divided into 4 different groups with one female and one male mouse in each group.

Groups 1 and 2 received pre-treatment with doxorubicin liposomes (8.1mg/kg) by i.v. injection, four days and one day, respectively, before the main treatment with liposomal ²²³Ra (500kBq/kg ²²³Ra and doxorubicin liposomes 0.9mg/kg). Group 3 received the pre-treatment and the treatment simultaneously and group 4 got no pre-treatment.

Four hours after the treatment, blood was drawn by cardiac puncture under anesthesia (halothane), before euthanasia. The radioactivity in blood, urine, faeces and different tissues was measured in a Crystal II Multidetector System (Packard Inst. Co. Inc, Downers Grove, IL), and compared with measurements of injection standards from the test product.

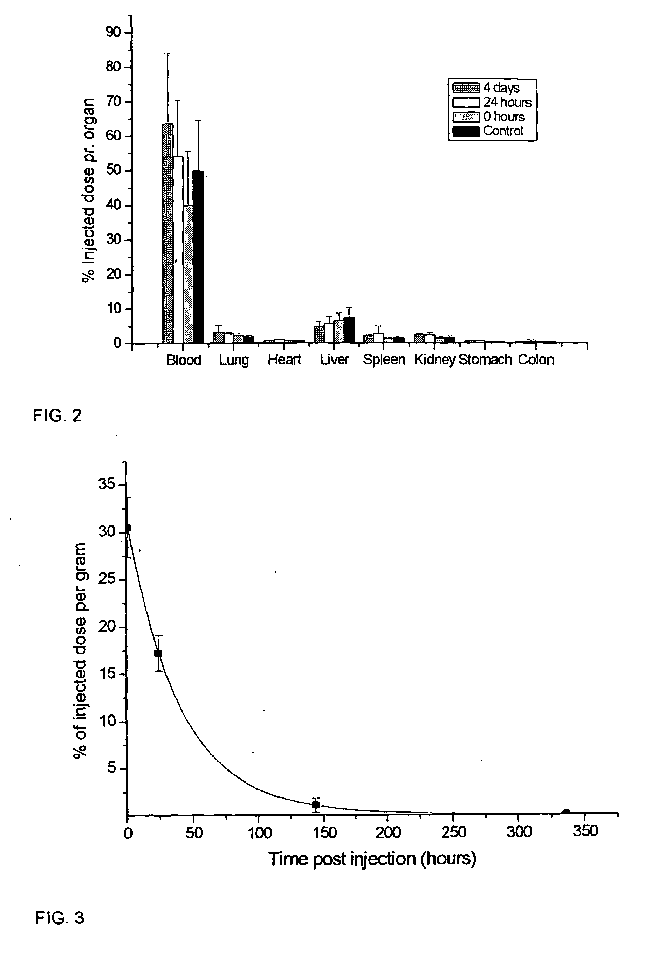

The data for the optimisation study are presented in Figure 2. An improved blood-to-liver ratio of liposomal ²²³Ra was achieved in animals pre-treated four days in advance with 8.1 mg per kg of Caelyx® vs. animals receiving pre-treatment one day in advance, co-injection or no pre-treatment with Caelyx®. Based on these results, a four days pre-treatment schedule was adopted for the main study.

### Example 4 Further biodistribution study with Pretreatment

Subsequently to Example 3, a more extensive biodistribution study was performed using the pre-treatment/treatment interval of four days, facilitating the highest blood/liver ratio of the liposomal ²²³Ra.

In the main study, the mice were divided into four different groups. Each group consisted of 6-7 animals of both sexes. Six mice in each group received an i.v. pre-treatment with doxorubicin liposomes (8.1mg/kg) four days before the main treatment with 375kBq/kg ²²³Ra and doxorubicin liposomes 0.9mg/kg iv. One mouse in each of groups two, three and four, were left untreated and were used to measure possible reuptake of the product from faeces or the bedding.

Blood was withdrawn, the mice were euthanised and the radioactivity measurements were performed in the same manner as in Example 3, 1 hour, 24 hours, 6 days and 14 days post injection for the respective groups of mice.

To study the difference between liposomally encapsulated and free ²²³Ra, a group of mice was injected intravenously with dissolved ²²³RaCl. Five animals were sacrificed at each of the time points 1 hour, 24 hours and 14 days post injection, and tissue samples were removed and radioactivity per weight unit determined as described for the study on liposomal ²²³Ra. The Localization Indices (LI) were calculated for liposomal vs free ²²³Ra by the following equation: LI = [% ID/g for liposomal ²²³Ra]/[% ID/g for free ²²³Ra] for a tissue.

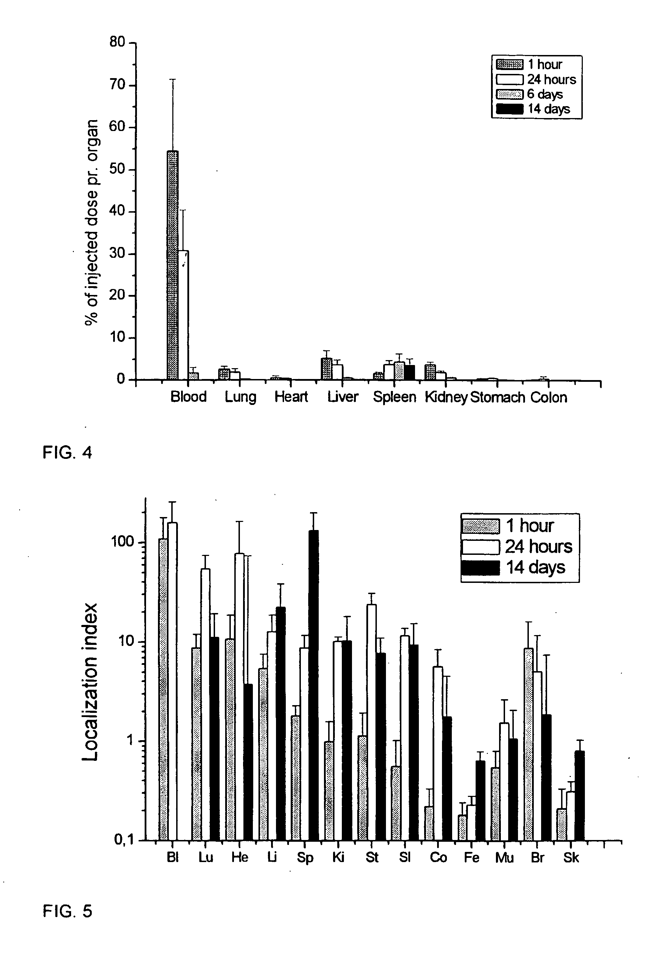

The data for the main biodistribution study is presented in Table 4 and Figure 3 and 4. The blood clearance of radioactivity (Figure 3) is consistent with clearance expected for this type of liposomes indicating that the liposomes are not destroyed by the bombardment of aplpha irradiation.

**Table 4 - Percent of injected dose per gram of tissue^{a} of liposomal ²²³Ra in Balb/C mice (main study) on four different time points.**

| Tissue | 1 hour | 24 hours | 6 days | 14 days |
|---|---|---|---|---|
| Blood (Bl) | 30.51 ± 3.19 | 17.21 ± 1.88 | 1.08 ± 0.77 | 0.05 ± 0.02 |
| Lung (Lu) | 11.89 ± 2.17 | 8.37 ± 1.63 | 1.29 ± 0.58 | 0.19 ± 0.10 |
| Heart (He) | 3.85 ± 2.54 | 2.85 ± 0.59 | 0.38 ± 0.25 | ND^{b} |
| Liver (Li) | 4.59 ± 0.8 | 3.19 ± 0.37 | 0.53 ± 0.07 | 0.17 ± 0.04 |
| Spleen (Sp) | 13.78 ± 2.32 | 32.36 ± 7.04 | 42.46 ± 16.92 | 29.47 ± 12.16 |
| Kidney (Ki) | 17.41 ± 2.34 | 9.07 ± 0.87 | 3.11 ± 0.82 | 0.53 ± 0.39 |
| Stomach (St) | 1.90 ± 0.29 | 2.50 ± 0.52 | 0.88 ± 0.24 | 0.32 ± 0.12 |
| Small intestine (Sl) | 2.22 ± 0.25 | 2.12 ± 0.24 | 0.39 ± 0.12 | 0.06 ± 0.02 |
| Large intestine (Co) | 1.27 ± 0.20 | 2.97 ± 1.04 | 0.63 ± 0.11 | 0.09 ± 0.04 |
| Muscle (Mu) | 0.61 ± 0.13 | 0.46 ± 0.15 | 0.26 ± 0.14 | 0.18 ± 0.15 |
| Brain (Br) | 0.71 ± 0.21 | 0.42 ± 0.15 | ND | ND |
| Skull (Sk) | 4.31 ± 1.55 | 6.38 ± 1.25 | 15.05 ± 4.56 | 12.38 ± 3.57 |
| Femur (Fe) | 5.82 ± 1.73 | 7.63 ± 0.74 | 15.63 ± 3.90 | 16.99 ± 3.85 |
| Skin | 0.69 ± 0.55 | 1.96 ± 1.14 | ND | ND |

| | | | | |
|---|---|---|---|---|
| ^{a}Mean ± SD, n = 5-6 animals ^{b}ND = not determined (because of low activity counts vs background counts) | | | | |

Among the soft tissues, the highest uptake per organ was observed in the liver (Figure 4). The weight adjusted liver uptake, on the other hand, appeared to be relatively low for the whole period studied while there was a significant uptake in the spleen when data are presented as % of injected dose per gram (Table 4). The uptake into the skeleton, as reflected by the activity in femur and skull, was increasing with time, probably because of a slowly catabolism of liposomes causing the release of ²²³Ra. Although liposomes could be expected to have some uptake in the bone marrow, which is present in the femur, the uptake in skull, which do not contain significant amount of bone marrow, indicate that most of the bone uptake is from free radium. It should be noted that the difference in uptake in femur vs. skull also was observed with cationic radium and does not necessarily reflect accumulation in the bone marrow.

In Figure 5 the Localization Indices (LI) in various tissues for liposomal radium vs. cationic radium at I hour, 24 hours and 14 days after injection are presented. Distinct differences between the two ²²³Ra forms were observed. The LI is particularly high for blood, partly because of the slow clearance of the liposomal radium from blood and partly due to the rapid clearance of free ²²³Ra. Also for most soft tissues, the LI's are significantly elevated. The LI's of the bone samples were less than 1 indicating a significant control of the biodistribution by the liposomal formulation, supressing the natural behaviour of radium as a calcium analogue and bone-seeker.

The half-life (T_{1/2}) for the liposomal formulation circulating in the blood was greater than 24 hours while the blood half-life of simple cationic ²²³Ra was much less than I hour.

### Example 5 further study of tumour targeting

Further study of pegylated dual-loaded liposomes to target solid tumors by exploiting the capillary leakage properties of tumor neovasculature. The goal of the study was to investigate distribution and tumor targeting properties of the alpha-particle emitter ²²³Ra, encapsulated in doxorubicin-containing-liposomes (Caelyx®/Doxil®).

Caelyx® was given before the injection of liposomal ²²³Ra to reduce the reticulo-endothelial-system uptake, as optimised in Example 3. Distribution and tumor uptake was evaluated in a human osteosarcoma xenograft mice model and in a dog with spontaneous osteosarcoma.

Blood clearance of the combination therapy was relatively slow, in mice t_{1/2} was ~28 h (BalbC mice) and in the dog t_{1/2} was - 39 h. In mice the liver uptake appeared to be relatively low in contrast to the spleen, where there was a significant uptake. In the dog the uptake in both liver and spleen was moderate.

In the xenograft model there was generally a higher retention of activity in the tumor vs. soft tissue. In the dog the 24 h uptake was considerably higher in both calcified and non-calcified tumor metastases of different organs, than in normal tissue.

Liposomal ²²³Ra combination therapy had a relevant biodistribution and blood clearance for tumor targeting and in particular showed favourable tumor/normal-tissue ratio in a dog with spontaneous osteosarcoma.

## Claims

1. A cytotoxic agent comprising liposomes wherein said liposomes enclose a solution comprising at least one alpha emitting radionuclide or at least one indirect alpha emitter which decays by beta-emission to form an alpha emitter and at least one other therapeutic agent, wherein said therapeutic agent is doxorubicin, wherein both the at least one alpha emitting radionuclide or at least one indirect alpha emitter and the at least one other therapeutic agent are cytotoxic and wherein said alpha emitting radionuclide or said indirect alpha emitter is at least one of ²¹¹At, ²¹²Bi, ²¹²Pb, ²¹³Bi, ²²³Ra, ²²⁴Ra, ²²⁵Ac or ²²⁷Th.

2. A cytotoxic agent as claimed in claim 1 wherein the liposomes also enclose at least one ionophore.

3. A cytotoxic agent as claimed in claim 1 or 2 wherein the liposomes also enclose at least one chelating or complexing agent.

4. A cytotoxic agent as claimed in any of claims 1 to 3 wherein said alpha emitting radionuclide or said indirect alpha emitter and/or doxorubicin is present at below the minimum dose normally required for therapeutic, prophylactic and/or palliative effectiveness when used as a single therapy.

5. A cytotoxic agent as claimed in any of claims 1 to 4 wherein said alpha emitting radionuclide or said indirect alpha emitter and doxorubicin present are synergistic in respect of their dosages and/or side effects.

6. A cytotoxic agent as claimed in any of claims 1 to 5 wherein said liposomes comprise at least one lipid having a thermotropic transition temperature of 10-50°C.

7. A method for the synthesis of a cytotoxic agent as claimed in any of claims 1 to 6, said method comprising contacting liposomes enclosing a solution comprising at least one therapeutic agent other than an alpha emitting radionuclide or an indirect alpha emitter with a loading solution comprising at least one ionophore and at least one alpha emitting radionuclide or at least one indirect alpha emitter, wherein said therapeutic agent is doxorubicin, wherein both the at least one alpha emitting radionuclide or at least one indirect alpha emitter and the at least one other therapeutic agent are cytotoxic and wherein said alpha emitting radionuclide or said indirect alpha emitter is at least one of ²¹¹At, ²¹²Bi, ²¹²Pb, ²¹³Bi, ²²³Ra, ²²⁴Ra, ²²⁵Ac or ²²⁷Th.

8. A method as claimed in claim 7 wherein said contacting is carried out at above physiological temperature.

9. A pharmaceutical composition comprising at least one cytotoxic agent as claimed in any of claims 1 to 6 and at least one pharmaceutically tolerable carrier and/or excipient.

10. A cytotoxic agent as claimed in any of claims 1 to 6 for use in therapy.

11. Use of a cytotoxic agent as claimed in any of claims 1 to 6 in the manufacture of a medicament for use in a method of treatment of hyperplastic or neoplastic disease.

12. The use as claimed in claim 11 wherein said treatment comprises administration of said medicament by systemic or intratumoral injection or infusion.

13. The use as claimed in claim 11 wherein said treatment comprises administration of said medicament by direct application to at least a part of a body cavity following resection of at least a part of a solid tumour.

14. The use as claimed in claim 11 or claim 12 wherein said treatment comprises pre-administration of at least one dose of non-radioactive liposomes, preferably 12 hour to 10 days before administration of said cytotoxic agent.

## Patentansprüche

1. Zytotoxikum, das Liposomen umfasst, wobei die Liposomen eine Lösung umschließen, die mindestens ein Alphastrahlungs-Radionuklid oder mindestens einen indirekten Alphastrahler, der durch Betastrahlung so zerfällt, dass er einen Alphastrahler ausbildet, und mindestens ein weiteres Therapeutikum umfasst, wobei das Therapeutikum Doxorubicin ist, wobei sowohl das mindestens eine Alphastrahlungs-Radionuklid oder der mindestens eine indirekte Alphastrahler als auch das mindestens eine weitere Therapeutikum zytotoxisch sind und wobei das Alphastrahlungs-Radionuklid oder der indirekte Alphastrahler mindestens eines der folgenden ist: ²¹¹At, ²¹²Bi, ²¹²Pb, ²¹³Bi, ²²³Ra, ²²⁴Ra, ²²⁵Ac oder ²²⁷Th.

2. Zytotoxikum nach Anspruch 1, wobei die Liposomen auch mindestens einen lonophor umschließen.

3. Zytotoxikum nach Anspruch 1 oder 2, wobei die Liposomen auch mindestens einen Chelat- oder Komplexbildner umschließen.

4. Zytotoxikum nach einem der Ansprüche 1 bis 3, wobei das Alphastrahlungs-Radionuklid oder der indirekte Alphastrahler und/oder Doxorubicin mit weniger als der Minimaldosis vorhanden ist/sind, die bei ihrer Verwendung als Monotherapie normalerweise für die therapeutische, prophylaktische und/oder palliative Wirksamkeit erforderlich ist.

5. Zytotoxikum nach einem der Ansprüche 1 bis 4, wobei das Alphastrahlungs-Radionuklid oder der indirekte Alphastrahler und Doxorubicin, die vorhanden sind, bezüglich ihrer Dosierungen und/oder Nebenwirkungen synergistisch sind.

6. Zytotoxikum nach einem der Ansprüche 1 bis 5, wobei die Liposomen mindestens ein Lipid mit einer thermotropen Übergangstemperatur von 10-50°C umfassen.

7. Verfahren zur Synthese eines Zytotoxikums nach einem der Ansprüche 1 bis 6, wobei das Verfahren umfasst: Inkontaktbringen von Liposomen, die eine Lösung umschließen, die mindestens ein Therapeutikum umfasst, bei dem es sich nicht um ein Alphastrahlungs-Radionuklid oder einen indirekten Alphastrahler handelt, mit einer Beladelösung, die mindestens einen Ionophor und mindestens ein Alphastrahlungs-Radionuklid oder mindestens einen indirekten Alphastrahler umfasst, wobei das Therapeutikum Doxorubicin ist, wobei sowohl das mindestens eine Alphastrahlungs-Radionuklid oder der mindestens eine indirekte Alphastrahler als auch das mindestens eine weitere Therapeutikum zytotoxisch sind und wobei das Alphastrahlungs-Radionuklid oder der indirekte Alphastrahler mindestens eines der folgenden ist: ²¹¹At, ²¹²Bi, ²¹²Pb, ²¹³Bi, ²²³Ra ²²⁴Ra_{,} ²²⁵Ac oder ²²⁷Th.

8. Verfahren nach Anspruch 7, wobei das Inkontaktbringen oberhalb der physiologischen Temperatur erfolgt.

9. Pharmazeutische Zusammensetzung, die mindestens ein Zytotoxikum nach einem der Ansprüche 1 bis 6 und mindestens ein(en) pharmazeutisch tolerierbaren/s Träger und/oder Bindemittel umfasst.

10. Zytotoxikum nach einem der Ansprüche 1 bis 6 zur therapeutischen Verwendung.

11. Verwendung eines Zytotoxikums nach einem der Ansprüche 1 bis 6 bei der Herstellung eines Medikaments zur Verwendung bei einem Behandlungsverfahren für eine Hyperplasie oder Neoplasie.

12. Verwendung nach Anspruch 11, wobei die Behandlung die Verabreichung des Medikaments durch systemische oder intratumorale Injektion oder Infusion umfasst.

13. Verwendung nach Anspruch 11, wobei die Behandlung die Verabreichung des Medikaments durch direkte Aufbringung auf zumindest einen Teil einer Körperhöhle nach der Resektion zumindest eines Teils eines festen Tumors umfasst.

14. Verwendung nach Anspruch 11 oder Anspruch 12, wobei die Behandlung die Vorab-Verabreichung mindestens einer Dosis nicht radioaktiver Liposomen, vorzugsweise 12 Stunden bis 10 Tage vor der Verabreichung des Zytotoxikums, umfasst.

## Revendications

1. Agent cytotoxique comprenant des liposomes dans lequel lesdits liposomes renferment une solution comprenant au moins un radionucléide émetteur de rayons alpha ou au moins un émetteur indirect de rayons alpha qui se désintègre par émission bêta pour former un émetteur de rayons alpha et au moins un autre agent thérapeutique, dans lequel ledit agent thérapeutique est la doxorubicine, dans lequel à la fois l'au moins un radionucléide émetteur de rayons alpha ou l'au moins un émetteur indirect de rayons alpha et l'au moins un autre agent thérapeutique sont cytotoxiques et dans lequel ledit radionucléide émetteur de rayons alpha ou ledit émetteur indirect de rayons alpha est au moins l'un de ²¹¹At, ²¹²Bi, ²¹²Pb, ²¹³Bi, ²²³Ra, ²²⁴Ra, ²²⁵Ac ou ²²⁷Th.

2. Agent cytotoxique selon la revendication 1, dans lequel les liposomes renferment également au moins un ionophore.

3. Agent cytotoxique selon la revendication 1 ou 2, dans lequel les liposomes renferment également au moins un agent chélatant ou complexant.

4. Agent cytotoxique selon l'une quelconque des revendications 1 à 3, dans lequel ledit radionucléide émetteur de rayons alpha ou ledit émetteur indirect de rayons alpha et/ou la doxorubicine est présent(e)/sont présents à un niveau inférieur à la dose maximale normalement requise pour l'efficacité thérapeutique, prophylactique et/ou palliative dans le cadre d'une monothérapie.

5. Agent cytotoxique selon l'une quelconque des revendications 1 à 4, dans lequel ledit radionucléide émetteur de rayons alpha ou ledit émetteur indirect de rayons alpha et la doxorubicine présents sont synergiques en ce qui concerne leurs dosages et/ou leurs effets secondaires.

6. Agent cytotoxique selon l'une quelconque des revendications 1 à 5, dans lequel lesdits liposomes comprennent au moins un lipide ayant une température de transition thermotrope de 10 à 50°C.

7. Procédé de synthèse d'un agent cytotoxique selon l'une quelconque des revendications 1 à 6, ledit procédé comprenant la mise en contact des liposomes renfermant une solution comprenant au moins un agent thérapeutique autre qu'un radionucléide émetteur de rayons alpha ou qu'un émetteur indirect de rayons alpha avec une solution de charge comprenant au moins un ionophore et au moins un radionucléide émetteur de rayons alpha ou au moins un émetteur indirect de rayons alpha, dans lequel ledit agent thérapeutique est la doxorubicine; dans lequel à la fois l'au moins un radionucléide émetteur de rayons alpha ou l'au moins un émetteur indirect de rayons alpha et l'au moins un autre agent thérapeutique sont cytotoxiques et dans lequel ledit radionucléide émetteur de rayons alpha ou ledit émetteur indirect de rayons alpha est au moins l'un de ²¹¹At, ²¹²Bi, ²¹²Pb, ²¹³Bi, ²²³Ra, ²²⁴Ra, ²²⁵Ac ou ²²⁷Th.

8. Procédé selon la revendication 7, dans lequel ladite mise en contact est effectuée à une température supérieure à la température physiologique.

9. Composition pharmaceutique comprenant au moins un agent cytotoxique selon l'une quelconque des revendications 1 à 6 et au moins un support et/ou excipient pharmaceutiquement acceptable.

10. Agent cytotoxique selon l'une quelconque des revendications 1 à 6, utilisable dans le cadre d'une thérapie.

11. Utilisation d'un agent cytotoxique selon l'une quelconque des revendications 1 à 6 dans la fabrication d'un médicament utilisable dans un procédé de traitement d'une maladie hyperplasique ou néoplasique.

12. Utilisation selon la revendication 11, dans laquelle ledit traitement comprend l'administration dudit médicament par injection ou perfusion par voie systémique ou intratumorale.

13. Utilisation selon la revendication 11, dans laquelle ledit traitement comprend l'administration dudit médicament par application directe sur au moins une partie d'une cavité corporelle puis par résection d'au moins une partie d'une tumeur solide.

14. Utilisation selon la revendication 11 ou 12, dans laquelle ledit traitement comprend l'administration préalable d'au moins une dose de liposomes non radioactifs, de préférence 12 heures à 10 jours avant l'administration dudit agent cytotoxique.
